Europäisches Patentamt

European Patent Office

Office européen des brevets

(11) Publication number : **0 566 333 A1**

# EUROPEAN PATENT APPLICATION

(21) Application number : 93302807.8

(51) Int. Cl.⁵ : **G01N 33/50, G01N 33/86**

(22) Date of filing : 08.04.93

(30) Priority : **13.04.92 US 868008**

(43) Date of publication of application :
**20.10.93 Bulletin 93/42**

(84) Designated Contracting States :
**AT BE CH DE DK ES FR GB IE IT LI LU MC NL PT SE**

(71) Applicant : **ORTHO DIAGNOSTIC SYSTEMS INC.**
**U.S. Route 202**
**Raritan New Jersey 08869 (US)**

(72) Inventor : **Moore, Bryant M.**
**6 Cyndi Court**
**Flemington, New Jersey 08822 (US)**
Inventor : **Hughes, James E.**
**P.O. Box 347**
**Reeders, PA 18352 (US)**
Inventor : **Venturini, Albert**
**RD No.1, Box 1038**
**East Stroudsburg, PA 18301 (US)**

(74) Representative : **Mercer, Christopher Paul et al**
**Carpmaels & Ransford 43, Bloomsbury Square**
**London WC1A 2RA (GB)**

(54) **Synthetic phospholipid reagent.**

(57)    The invention is directed to a synthetic reagent comprising phosphatidylethanolamine, phosphatidylserine and phosphatidylcholine. A method of using the phospholipid reagents in determining clotting time of a sample is also provided.

EP 0 566 333 A1

Jouve, 18, rue Saint-Denis, 75001 PARIS

## Field of the Invention

The invention relates to synthetic phospholipid reagents and a method of using the reagents in diagnostic assays such as an Activated Partial Thromboplastin Time test, or a Prothrombin Time test, to determine blood clotting times.

## Background of the Invention

Screening tests for coagulation disorders include the activated partial thromboplastin time (APTT) and the prothrombin time (PT). Essentially, screening tests for coagulation disorders are designed to detect a significant abnormality in one or more of the clotting factors and to localize this abnormality to various steps in the coagulation pathway.

The clinical value of testing of coagulation time is not limited to the detection of genetic or pathological disease states, but is also useful in the regulation of anticoagulation therapy. Anticoagulants typically inhibit the coagulation mechanism such as by the heparin mediated inhibition of Factor X by Anti-Thrombin III or through the action of coumadin (warfarin) which inhibits carboxylation of the Vitamin K dependent Factors II, VII, X, XI, Protein C and Protein S.

As commonly understood, coagulation may occur by two pathways, the intrinsic pathway and the extrinsic pathway. The former is generally triggered by the presence of a surface, (thought to activate Factor XII) and with the presence of phospholipids and calcium, through a number of steps eventually stimulates the formation of a stabilized fibrin clot. The APTT test typically measures coagulation factors of the intrinsic pathway, where most congenital deficiencies occur, and the PT test measures coagulation factors of the extrinsic pathway.

The Activated Partial Thromboplastin Time, APTT, test is a diagnostic test employed in the evaluation of the time of blood to clot. It is used to determine the presence of and quantitation of various coagulation factors, Factors XII, XI, IX, VIII, X, V, II and I, as they relate to diseased states which display abnormal blood clotting mechanisms. Thus, the APTT test is useful as a presurgical screen and for monitoring heparin therapy.

The APTT test is performed by adding an activator such as kaolin, silica, ellagic acid, and the like with phospholipid based reagent to plasma. This activates Factors XII and XI. Currently in the APTT, the phospholipids employed are extracted from bovine or rabbit brain, although sources such as soy bean have been used. The exogenous phospholipids of the APTT reagent substitute for the phospholipids provided by platelets *in vivo* in the activation of Factor VIII by Factors IX, VIII and V. Blood coagulation is initiated in this clotting test by adding calcium. Factor VII is the only factor not affected by the partial thromboplastin time. Thus, the APTT is, therefore, normal in patients with a Factor VII deficiency.

The prothrombin time (PT) test is performed by adding tissue thromboplastin with calcium to plasma. This initiates clotting by activating Factor VII which in turn activates Factor X which in the presence of Factor V, leading to the conversion of prothrombin to thrombin. The thrombin which is so produced converts fibrinogen to fibrin. PT therefore bypasses the intrinsic clotting pathway and is normal in patients with deficiencies of Factors XII, XI, IX and VIII. PT is abnormal in patients with deficiencies of Factors VII, X, V, prothrombin or fibrinogen. Tissue thromboplastin is a phospholipid extract (from rabbit brain or lung and human brain or placenta) to which calcium has been added. It is usually provided in a lyophilized form and must be reconstituted with distilled water.

The APTT and PT have found wide acceptance despite the fact that natural phospholipid reagents employed in the tests are inconsistent as to source and quantity of the individual phospholipids. One drawback to the use of the natural phospholipid extracts as reagents for diagnostic assays is the inconsistent nature of the total phospholipid content between sources, as well as the ratio of individual phospholipids contained in the extracts. The phospholipids from these natural sources differ in the ratio of each phospholipid type present; phosphatidylcholine, phosphatidylethanolamine, phosphatidylinositol, phosphatidylserine. There is also great variability in the degree of unsaturation and length of the respective fatty acyl chains depending on the natural source of the respective phospholipids.

Accordingly, there remains a need for synthetic, purified phospholipid reagents for use in diagnostic assays which exhibit control and consistency in manufacture, offer the potential to alter ratios of individual phospholipids, insure more reliability in and improved sensitivity to the determination of different coagulation factors, and eliminate assay performance variability caused by variability in content of individual phospholipids and heterogeneous fatty acyl chain structure.

## Summary of the Invention

The invention is directed to a synthetic, purified reagent comprising phosphatidylethanolamine, phosphatidylserine and phosphatidylcholine. A method of using the synthetic phospholipid reagents in diagnostic assays such as determining clotting time of a sample is also provided.

## Detailed Description of the Invention

The synthetic mixture of phospholipids of the invention are used to replace the use of naturally extracted phospholipids currently employed in laboratory assays. The synthetic phospholipids of the claimed invention are manufactured and purified from chemical sources, and are known to those of ordinary skill in the art. Thus, the synthetic phospholipid reagent of the claimed invention differs from the phospholipid reagents currently available which are isolated from natural sources including brain and soybean and which are provided by manufacturers of these reagents. The synthetic mixture of phospholipids includes phosphatidylcholine, phosphatidylethanolamine and phosphatidylserine. These synthetic phospholipids have well defined ratios and identical fatty acyl chain structure. Added together in the proper ratio, these synthetic phospholipids form a mixture which should perform equal to or better than their naturally occurring counterparts. The synthetic reagents also produce more consistent results and have improved assay performance.

The invention also includes a method of using the purified phospholipids in determining clotting time of a sample. The activated partial thromboplastin time (APTT) and prothrombin time (PT) tests are used to indicate abnormalities in most of the procoagulant clotting factors. The APTT is a useful sensitive procedure for generating heparin response curves and for screening deficiencies of clotting factors in the intrinsic pathway, one of two coagulation pathways. The PT is a useful test to determine deficiencies of clotting factor activity in the extrinsic pathway.

As contemplated in the method of this invention, a synthetic phospholipid reagent is combined with test plasma and clotting time is determined. The test plasma may be deficient in a specific intrinsic factor, such as Factor VIII, IX, XI, XII and the like, or a specific extrinsic factor, such as Factor VI, X, V, prothrombin, fibrinogen and the like. The test plasma may also be lupus deficient, heparin deficient, or the like, for example. The clotting reaction is initiated by the addition of ionic calcium and the test result is the time required for clot formation.

The APTT test encompasses all three stages of coagulation and thereby indicates an abnormality in most of the procoagulant clotting factors if one exists. The APTT test is a useful sensitive procedure for generating heparin response curves and for screening deficiencies of clotting factors in the intrinsic pathway of coagulation with the exception of platelet factor III. Initially, phospholipid reagent is mixed with the test plasma. After incubation at 37°C for a specific period of time, the reaction is initiated by the addition of ionic calcium. The time, in seconds, required for clot formation is the test result. An extended APTT result is usually indicative of a decreased level of one or more of the coagulation factors in the intrinsic pathway or the presence of an anticoagulant like heparin.

The prothrombin time, PT, test is used to determine deficiencies of clotting factor activity, either hereditary or acquired, in the extrinsic pathway. The extrinsic pathway includes plasma coagulation factors VI, X, V, prothrombin and fibrinogen. Although the extrinsic pathway also includes factor III (tissue thromboplastin), it is not measured in the PT test. Since most of the factors depressed by oral anticoagulant drugs are in the extrinsic pathway, PT is the test of choice in controlling oral anticoagulant therapy.

Tissue thromboplastin, in the presence of calcium, is an activator which initiates the extrinsic pathway of coagulation. By adding tissue thromboplastin to normal anticoagulated plasma, the clotting mechanism is initiated and a fibrin clot will form within a specified period of time. If there is a deficiency of factor activity in the extrinsic pathway of coagulation, the time required for formation of the clot will be prolonged beyond that expected for normal plasma. The results that follow indicate that as phospholipid concentration in an assay reagent increases, clotting times for various samples is expected to increase or decrease depending on the specific phospholipid and concentrations employed, see TABLE I, which is a summary of TABLES II and III. The samples include normal (OPCCI) and abnormal (OPCCII & OPCCIII) controls, plasma deficient samples including Factor VIII & IX (F VIII, F IX) lupus and heparin deficient samples.

TABLE I

| As Phospholipid Concentration Increases | | | |
|---|---|---|---|
| Clotting Time | PS | PE | PC |
| OPCCI | increases | decreases | decreases |
| OPCCII | increases | decreases | decreases |
| OPCCIII | increases | decreases | decreases |
| F VIII | increases | increases | decreases |
| F IX | increases | decreases | decreases |
| LUPUS | increases | increases | decreases |
| HEPARIN | increases | decreases | decreases |

The synthetic phospholipid reagent of the claimed invention is a buffered reagent which provides controlled phospholipid concentrations and constant activation of the contact system with a soluble, nonsettling activator. Since soluble activators do not precipitate out of solution, they provide uniform activation of the plasma specimen making the test results more reproducible.

The synthetic phospholipid reagent comprises a mixture of phosphatidylethanolamine, phosphatidylcholine, phosphatidylserine, a buffer such as N-2-hydroxyethylpiperazine-N'-2-ethanesulfonic acid and the like, a stabilizer such as amino acids, dextrose and the like, and a preservative such as thimerosal (for example 0.01%), neomycin sulfate, and phenol mazide. Each component is present in the mixture in the following concentrations: stabilizer - less than or equal to 5.0%, more preferably greater than or equal to 0.5%, most preferably about 0.5% to about 5.0%; buffer - less than or equal to 1.0 M, more preferably greater than or equal to 0.09 M, most preferably about 0.09 to about 1.0 M; and preservative - less than or equal to 0.6%, more preferably greater than or equal to 0.01% and most preferably about 0.01% to about 0.6%.

The present invention demonstrates the feasibility of using synthetic phospholipids of a chemical source in place of phospholipids derived from animal tissue or plant sources to act as platelet-like substitute in a coagulation assay reagent, using ORTHO Diagnostic Thrombosil as a reagent model system control, see TABLE III, column "CURRENT TSIL".

The invention is further illustrated by means of the following example. The example is meant to be illustration only and is not intended to limit the present invention to the specific embodiments.

**Example**

The computerized response surface (face centered cube) experimental screening design was used to demonstrate the significant effects of the individual phospholipids. The interaction of phospholipid ratios on performance characteristics (as clotting time, in seconds) using normal and specific factor deficient plasmas was demonstrated.

Minimum and maximum ranges (in µg/ml) of phosphatidylserine (PS), phosphatidylethanolamine (PE) and phosphatidylcholine (PC) to incorporate into a synthetic reagent were determined by measuring the concentrations of each phospholipid in currently available Ortho and Competitive APTT reagents by HPLC. Surface response screening design resulted in 17 experimental synthetic formulations.

These 17 experimental synthetic formulations, each formulation having a different PS:PE:PC ratio, were then assayed in surface response tests to predict clotting times of each formulation, see TABLE II. Each formulation was tested against control samples including plasma samples deficient in PS, PE or PC, lyophilized normal plasma sample (OPCCI), lyophilized abnormal plasma samples (OPCCII, low abnormal and OPCCIII, high abnormal), see TABLE II, columns A-F. The formulations were also tested against lyophilized plasma deficient samples, see TABLE II, columns G-M, including Factor VIII, IX, XI and XII, heparin from normal plasma pool, (HEP0U-0 U/ml, HEP2U-0.2 U/ml) and lupus deficient sample, Lupus control plasma (aracknase). Difference in phospholipid levels of PS, PE and PC, as well as the total stability of PS, PE and PC mixture, at 4 week intervals were also tested, see TABLE II, columns N-Q.

## TABLE II

## SYNTHETIC APTT

Controlled Factors

| RUN | A PS | B PE | C PC | D OPCCI | E OPCCII | F OPCCIII | G F8 | H F9 |
|-----|------|------|------|---------|----------|-----------|------|------|
| 1 | 43.00 | 140.5 | 72.5 | 26.40 | 45.20 | 59.90 | 135.30 | 138.50 |
| 2 | 43.00 | 27.0 | 72.5 | 27.10 | 51.00 | 70.80 | 143.30 | 137.50 |
| 3 | 43.00 | 140.5 | 72.5 | 29.60 | 46.40 | 62.80 | 135.70 | 137.70 |
| 4 | 75.00 | 254.0 | 17.0 | 26.60 | 44.30 | 59.00 | 122.80 | 132.30 |
| 5 | 43.00 | 140.5 | 72.5 | 25.40 | 45.30 | 60.70 | 130.70 | 131.90 |
| 6 | 75.00 | 27.0 | 128.0 | 27.20 | 52.50 | 74.60 | 144.20 | 145.30 |
| 7 | 11.00 | 254.0 | 128.0 | 26.30 | 51.40 | 71.80 | 102.40 | 104.10 |
| 8 | 43.00 | 254.0 | 72.5 | 25.60 | 48.70 | 66.70 | 129.40 | 129.80 |
| 9 | 11.00 | 27.0 | 128.0 | 38.30 | 83.20 | 107.50 | 150.00 | 150.00 |
| 10 | 75.00 | 27.0 | 17.0 | 34.20 | 61.30 | 88.30 | 129.70 | 141.40 |
| 11 | 11.00 | 254.0 | 17.0 | 27.80 | 46.50 | 61.70 | 124.10 | 125.30 |
| 12 | 75.00 | 140.5 | 72.5 | 26.40 | 48.60 | 67.10 | 118.60 | 132.70 |
| 13 | 75.00 | 254.0 | 128.0 | 26.20 | 50.50 | 70.30 | 124.60 | 132.00 |
| 14 | 11.00 | 27.0 | 17.0 | 28.20 | 48.00 | 63.80 | 128.10 | 131.80 |
| 15 | 11.00 | 140.5 | 72.5 | 27.00 | 49.20 | 67.20 | 112.50 | 113.40 |
| 16 | 43.00 | 140.5 | 128.0 | 27.90 | 55.50 | 77.70 | 137.40 | 136.00 |
| 17 | 43.00 | 140.5 | 17.0 | 27.30 | 46.90 | 62.90 | 123.80 | 129.20 |

Measured Characteristics

| I | J | K | L | M | N | O | P | Q |
|---|---|---|---|---|---|---|---|---|
| F11 | F12 | HEPOU | HEP2U | LUPUS | PSTAB | PESTAB | PCSTAB | TOTSTA |
| 131.80 | 150.0 | 28.60 | 44.60 | 62.60 | 2.00 | 5.0 | 5.00 | 12.0 |
| 136.30 | 150.0 | 28.90 | 51.80 | 60.00 | 12.00 | 4.0 | 4.00 | 20.0 |
| 132.70 | 150.0 | 28.10 | 46.80 | 58.30 | 9.00 | 17.0 | 2.00 | 28.0 |
| 132.80 | 150.0 | 28.40 | 44.60 | 58.40 | 59.00 | 110.0 | 10.00 | 179.0 |
| 127.90 | 150.0 | 26.90 | 45.40 | 58.60 | 10.00 | 16.0 | 13.00 | 39.0 |
| 144.00 | 150.0 | 29.00 | 53.60 | 59.90 | 1.00 | 0.0 | 0.00 | 0.0 |
| 135.80 | 150.0 | 27.70 | 51.20 | 76.40 | 2.00 | 15.0 | 5.00 | 22.0 |
| 134.80 | 150.0 | 27.60 | 49.60 | 59.20 | 4.00 | 15.0 | 3.00 | 22.0 |
| 150.00 | 150.0 | 43.50 | 90.00 | 127.00 | 2.00 | 4.0 | 3.00 | 10.0 |
| 146.70 | 150.0 | 35.00 | 63.40 | 74.60 | 9.00 | 0.0 | 1.00 | 10.0 |
| 130.60 | 150.0 | 30.40 | 48.80 | 58.50 | 4.00 | 10.0 | 4.00 | 18.0 |
| 133.00 | 150.0 | 30.10 | 46.50 | 50.00 | 40.00 | 50.0 | 28.00 | 218.0 |
| 137.10 | 150.0 | 30.10 | 50.20 | 51.40 | 49.00 | 141.0 | 34.00 | 225.0 |
| 134.90 | 150.0 | 30.60 | 49.20 | 61.20 | 3.00 | 8.0 | 1.00 | 12.0 |
| 133.20 | 150.0 | 29.70 | 52.00 | 73.60 | 3.00 | 3.0 | 0.00 | 5.0 |
| 143.20 | 150.0 | 31.30 | 56.80 | 63.70 | 8.00 | 1.0 | 8.00 | 17.0 |
| 133.50 | 150.0 | 30.90 | 47.80 | 54.70 | 0.00 | 5.0 | 0.00 | 4.0 |

Each experimental formulation was tested in random order on a commercially available Ortho instrument to obtain APTT clotting times. The data was then entered for computer analysis to determine quadratic regression analysis to show which factors or interaction of factors are most significant in influencing performance characteristics and residuals tables to demonstrate the correlation between observed and predicted results. The control sample, "CURRENT TSIL", is the currently commercially available Ortho reagent Thrombosil. As a result, 5 formulations of synthetic phospholipid reagent and their characteristics were determined as likely to be observed, see Table III. Concentrations ranges (in μg/ml) of each phospholipid in the five formulations were calculated. Phosphatidylserine concentration of less than or equal to 57.5 μg/ml, more prefereably greater than or equal to 11.0 μg/ml, and most preferably from about 11.0 to about 57.5 μg/ml was calculated. Phosphatidylethanolamine concentration of less than or equal to 254.0 μg/ml, more preferably greater than or equal to 27.0 μg/ml, and most preferably from about 27.0 to about 254.0 μg/ml was calculated. Phosphatidylcholine concentration of less than or equal to 128.0 μg/ml, more preferably greater than or equal to 17.0 μg/ml, and most preferably from about 17.0 to about 128.0 μg/ml was calculated. These concentration ranges for the phospholipid reagent formulations result in clotting time characteristics similar to that received with Thrombosil, see Table III.

## TABLE III

### SYNTHETIC SILICA APTT REAGENT

| FACTORS CHARACT. | FORM 1 | FORM 2 | FORM 3 | FORM 4 | FORM 5 | CURRENT TSIL |
|---|---|---|---|---|---|---|
| PHOSPHOLIPID μg/ml | | | | | | |
| PS | 11.00 | 34.50000 | 11.00 | 11.000 | 57.50000 | 101.0 |
| PE | 155.00 | 125.0000 | 27.00 | 254.000 | 75.60000 | 183.0 |
| PC | 110.00 | 121.00000 | 57.70 | 128.000 | 17.00000 | 133.0 |
| CLOTTING TIME CHARACTERISTICS: | | | | | | |
| OPCCI | 29.70 | 29.00000 | 30.40 | 28.800 | 29.60000 | 30.9 |
| OPCCII | 57.90 | 56.70000 | 57.40 | 57.800 | 50.20000 | 55.7 |
| OPCCIII | 77.60 | 76.70000 | 75.30 | 80.800 | 70.00000 | 83.7 |
| F8 | 115.00 | 135.00000 | 134.00 | 107.000 | 130.00000 | 117.0 |
| F9 | 119.00 | 135.00000 | 134.00 | 109.000 | 137.00000 | 121.8 |
| F11 | 137.00 | 140.00000 | 136.00 | 140.000 | 138.00000 | 130.8 |
| HEPOU/ML | 32.80 | 32.00000 | 33.10 | 30.700 | 31.20000 | 32.9 |
| HEP.2U/ML | 60.00 | 58.40000 | 60.20 | 69.200 | 50.80000 | 56.7 |
| LUPUS | 87.20 | 74.50000 | 82.40 | 84.600 | 58.20000 | 55.6 |
| PS STAB | 4.48 | 0.04160 | 8.66 | -0.952 | 6.12000 | 66.0 |
| PE STAB | 3.38 | 0.01990 | 13.60 | 10.700 | 0.00069 | 23.0 |
| PC STAB | 7.25 | 4.93000 | 4.71 | 5.140 | 0.10500 | 23.0 |
| TOTAL STAB | 37.10 | 0.00843 | 37.80 | 7.350 | 1.66000 | 112.1 |

Optimization tables in TABLE III were generated to show which way performance data moves with respect to increases or decreases in factors and interactions of factors.

Optimized levels of phospholipids and other reagent components required to yield the desired accuracy, precision and stability performance characteristics with respect to all APTT reagent applications were determined.

Performance criteria include normal patient/control plasmas (OPCCI) and abnormal patient/control plasmas including in vivo/in vitro heparinized plasma (HEP OU/ml, HEP 0.2U/ml), liver disease (OPCCII, OPCCIII), intrinsic factor deficiencies (F8, F9, F11), lupus anticoagulants (lupus) and abnormal controls (OPCCII, OPCCIII). Computerized experimental design software was utilized to design and analyze experimental formulations.

**Claims**

1. A reagent comprising a mixture of synthetic phosphatidylserine, phosphatidylethanolamine and phosphatidylcholine.

2. A reagent of claim 1 further including a buffer, a stabilizer and a preservative.

3. A reagent of claim 2, wherein said buffer is in a concentration of about 0.09 to 1.0 M, said stabilizer is in a concentration of about 0.5% to 5.0%, and said preservative is in a concentration of about 0.01% to 0.6%.

4. A reagent of claim 2 wherein said buffer is N-2-hydroxyethyl-piperazine-N'-2-ethanesulfonic acid.

5. A reagent of claim 2 wherein said preservative is thimerosal, neomycin sulfate or phenol mazide.

6. A reagent of claim 2 wherein said stabilizer is amino acids or dextrose.

7. A reagent of claim 1, wherein the concentrations, in µg/ml, of phosphatidylserine ranges of from about 11.0 to about 57.5, of phosphatidylethanolamine ranges of from about 27.0 to about 254.0 and of phosphatidylcholine of from about 17.0 to about 128.0.

8. A method of determining clotting time of a sample using the reagent of claim 1.

**European Patent Office**

# EUROPEAN SEARCH REPORT

Application Number

EP 93 30 2807

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 204 045 (BIO/DATA CORPORATION) <br> * claims 9,17 * <br> --- | 4-6 | G01N33/50 <br> G01N33/86 |
| A | WO-A-9 011 368 (G. J. PROKSCH) <br> --- | | |
| P,X | WO-A-9 208 479 (CORVAS INTERNATIONAL, INC.) <br> * the whole document * <br> ----- | 1-8 | |
| | | | **TECHNICAL FIELDS SEARCHED (Int. Cl.5)** |
| | | | G01N <br> C12Q |

The present search report has been drawn up for all claims

| Place of search | Date of completion of the search | Examiner |
|---|---|---|
| THE HAGUE | 14 JULY 1993 | CARTAGENA ABELLA P |

EPO FORM 1503 03.82 (P0401)

9